# EUROPÄISCHE PATENTSCHRIFT

(11) **EP 0 841 057 B1**
(45) Veröffentlichungstag und Bekanntmachung des Hinweises auf die Patenterteilung: **02.11.2006**
(21) Anmeldenummer: 97115975.1
(22) Anmeldetag: 13.09.1997
(51) Int. Cl.: A61K 8/55, A61K 8/97, A61Q 19/00, C09K 15/00, C11B 5/00, A23J 7/00, A23L 1/30

(54) **Phospholipidische Zusammensetzung, Verfahren zur Herstellung einer derartigen Zusammensetzung und Verwendung derselben**
Phospholipid composition, method of making it and its use
Composition phospholipidique, procédé de préparation d'une telle composition, et son utilisation

(30) Priorität: 06.11.1996 DE 19645657
(43) Veröffentlichungstag der Anmeldung: 13.05.1998
(73) Patentinhaber: Phospholipid GmbH, 50829 Köln (DE)
(72) Erfinder: Ghyczy, Miklos, Dr., 50933 Köln (DE)
(74) Vertreter: Lau-Loskill, Philipp

(56) Entgegenhaltungen:
- EP-A- 0 372 669
- WO-A-85/00746
- WO-A-95/20945
- US-A- 5 175 012
- DATABASE WPI Week 8901 Derwent Publications Ltd., London, GB; AN 89-004324 XP002108557 & JP 63 283735 A (NAKANO VINEGAR CO. LTD.) , 21. November 1988 (1988-11-21)
- PATENT ABSTRACTS OF JAPAN vol. 16, no. 213 (C-942), 20. Mai 1992 (1992-05-20) & JP 04 039397 A (KONGOU YAKUHIN KK), 10. Februar 1992 (1992-02-10)
- DATABASE WPI Week 7828 Derwent Publications Ltd., London, GB; AN 78-50607A XP002108558 & JP 53 062847 A (AJINOMOTO KK), 5. Juni 1978 (1978-06-05)
- DATABASE WPI Week 8004 Derwent Publications Ltd., London, GB; AN 80-06182c XP002108559 & JP 54 155205 A (CHURI GOSHI KAISHA), 7. Dezember 1979 (1979-12-07)

## Beschreibung

Die vorliegende Erfindung betrifft eine phospholipidische Zusammensetzung mit den Merkmalen des Oberbegriffs des Patentanspruchs 1, ein Verfahren zur Herstellung einer derartigen Zusammensetzung mit den Merkmalen des Oberbegriffs des Patentanspruchs 14 sowie die Verwendung der phospholipidischen Zusammensetzung.

Phospholipidische Zusammensetzungen sind seit langem bekannt und werden vielfach im Lebensmittelbereich, in der Tierernährung, im kosmetischen Bereich sowie im pharmazeutischen Bereich verwendet. Hierbei besteht jedoch das Problem, daß derartige phospholipidische Zusammensetzungen, die abhängig von der Konzentration an Phospholipiden und der jeweiligen Anwendung, flüssig, halbfest, wie insbesondere gelartig, cremeartig, pastös, oder fest sind, sehr leicht an der Luft und/oder beim Lagern einen Eigengeruch, insbesondere einen ranzigen Eigengeruch, entwickeln, was dementsprechend unerwünscht ist.

Um diese unerwünschte Geruchsveränderung von phospholipidischen Zusammensetzungen zu unterdrücken, ist es bekannt, die phospholipidischen Zusammensetzungen mit einem Stabilisator zu versehen, wobei derartige Stabilisatoren auf der Basis von Vitamin C, Vitamin E und/oder deren Derivaten aufgebaut sind. Desweiteren können phospholipidische Zusammensetzungen auch gemäß der DE 41 41 842 A1 durch Verwendung von N-Acyl-Phosphatidylethanolaminen vor einer unerwünschten Oxidation geschützt werden.

Darüber hinaus schlägt die DE 40 21 082 A1 vor, zur Stabilisierung von phospholipidischen Zusammensetzungen Harnstoff, Monosaccharide oder Mischungen von Harnstoff mit Monosacchariden zu verwenden.

Der vorliegenden Erfindung liegt die Aufgabe zugrunde, eine phospholipidische Zusammensetzung der angegebenen Art zur Verfügung zu stellen, die auch bei einer längeren Lagerung selbst unter Luftzufuhr keine Geruchsveränderung erfährt.

Diese Aufgabe wird erfindungsgemäß durch eine phospholipidische Zusammensetzung mit den kennzeichnenden Merkmalen des Patentanspruchs 1 gelöst.

Die erfindungsgemäße phospholipidische Zusammensetzung umfaßt mindestens ein Phospholipid sowie einen Stabilisator, wobei die Zusammensetzung neben dem mindestens einen Phospholipid desweiteren als Stabilisator zerkleinerte Bestandteile und/oder einen Extrakt von Getreide enthält.

Überraschend konnte festgestellt werden, daß die erfindungsgemäße phospholipidische Zusammensetzung, die als Stabilisator zerkleinerte Bestandteile und/oder einen Extrakt von Getreide enthält, eine hervorragende Stabilität selbst bei einer Lagerung unter Luft aufweist, so daß auch nach einer längeren Lagerzeit von mehreren Monaten keine unerwünschte Geruchsveränderung bei der so stabilisierten phospholipidischen Zusammensetzung auftritt. Dies führt dazu, daß die erfindungsgemäße phospholipidische Zusammensetzung nicht, wie die bisher bekannten phospholipidischen Zusammensetzungen, unter Inertgas, so zum Beispiel Stickstoff, bei verringerten Temperaturen, so insbesondere Temperaturen um den Gefrierpunkt, gelagert werden muß. Auch beeinflußt in der erfindungsgemäßen phospholipidischen Zusammensetzung der zuvor beschriebene Stabilisator die Konsistenz der phospholipidischen Zusammensetzung positiv, d.h. die ursprünglich für phospholipidische Zusammensetzungen, die einen hohen Gehalt an Phosphatidylcholin aufweisen, bekannte wachsartige Konsistenz, die sich in der Regel schon einige Minuten nach der Herstellung von derartigen phospholipidischen Zusammensetzungen einstellt, kann bei der erfindungsgemäßen Zusammensetzung nicht beobachtet werden. Vielmehr bleibt die erfindungsgemäße Zusammensetzung, abhängig von ihrem Herstellungsverfahren, pulvrig oder granuliert, so daß die Weiterverarbeitung und Handhabung der erfindungsgemäßen Zusammensetzung im Vergleich zu den üblichen, wachsartigen phospholipidischen Zusammensetzungen erheblich vereinfacht ist. Dementsprechend lassen sich aus der erfindungsgemäßen Zusammensetzung auch bezüglich der Phospholipidkonzentration reproduzierbare Folgeprodukte, wie beispielsweise Creme, Gele, Salben, Dispersionen, halbfeste oder flüssige liposomale Zubereitungen, herstellen, da die granulierte bzw. pulverisierte erfindungsgemäße Zusammensetzung wesentlich einfacher und genauer dosiert bzw. abgemessen werden kann.

Bei flüssigen phospholipidischen Zusammensetzungen bewirkt der zuvor genannte Stabilisator, daß diese flüssigen phospholipidischen Zusammensetzungen gegen einen chemischen Abbau hervorragend stabilisiert werden, so daß dementsprechend keine Veränderung des chemischen Aufbaus der darin enthaltenen Phospholipide auftritt. Dies wiederum führt dazu, daß derartige flüssige phospholipidische Zusammensetzungen auch nach einer längeren Lagerung eine weitestgehend konstante Viskosität aufweisen, so daß dementsprechend diese flüssigen phospholipidische Zusammensetzungen genau und reproduzierbar dosierbar sind.

Eine erste Ausführungsform der erfindungsgemäßen Zusammensetzung sieht vor, daß die Zusammensetzung als Stabilisator zerkleinerte Bestandteile und/oder ein Extrakt der Samenschalen und/oder der Aleuronschicht von Getreide enthält. Somit weist diese Ausführungsform der erfindungsgemäßen Zusammensetzung solche zerkleinerten Getreidebestandteile bzw. Getreideextrakte auf, die auch üblicherweise als zerkleinerte Kleiebestandteile und/oder als Kleieextrakte zu bezeichnen sind.

Vorzugsweise enthält die erfindungsgemäße phospholipidische Zusammensetzung als Stabilisator solche zerkleinerten Kleiebestandteile und/oder solche Kleieextrakte auf, die durch die Zerkleinerung der Samenschalen und/oder der Aleuronschicht und/oder die durch Extraktion, vorzugsweise durch Extraktion mit Wasser oder einem wäßrigen System, der Samenschalen und/oder der Aleuronschicht von Weizen, Hafer, Mais, Reis, Roggen, Gerste und/oder Hirse gewonnen werden.

Unter Getreide im Rahmen der vorliegenden Anmeldung werden alle diejenigen Gräser verstanden, die wegen ihrer eßbaren Früchte angebaut werden.

Bezüglich des Gewichtsverhältnisses von dem mindestens einen Phospholipid zu den zerkleinerten Bestandteilen des Getreides bzw. zu den zerkleinerten Bestandteilen der Samenschalen und/oder der Aleuronschicht von Getreide in der erfindungsgemäßen Zusammensetzung ist festzuhalten, daß sich dieses Gewichtsverhältnis einerseits nach der gewünschten Stabilisierung und andererseits nach der erwünschten Festigkeit und Konsistenz der granulierten bzw. pulverisierten erfindungsgemäßen phospholipidischen Zusammensetzung richtet. Üblicherweise variiert in der erfindungsgemäßen Zusammensetzung das Gewichtsverhältnis von dem mindestens einen Phospholipid zu dem zuvor beschriebenen Stabilisator zwischen 9:1 und 1:4, vorzugsweise zwischen 6:1 und 1:2. Ist ein relativ festes, besonders haltbares Granulat bzw. Pulver der erfindungsgemäßen Zusammensetzung erwünscht, so liegt hierbei vorzugsweise das Gewichtsverhältnis von dem mindestens einen Phospholipid und dem zuvor beschriebenen Stabilisator zwischen 1:1 und 1:4, während für weichere Granulate bzw. Pulver der erfindungsgemäßen Zusammensetzung das Gewichtsverhältnis von dem mindestens einen Phospholipid zu dem zuvor beschriebenen Stabilisator zwischen 9:1 und 1:1 variiert. Mit anderen Worten läßt sich somit durch Variation der Menge des zuvor benannten Stabilisators eine gewünschte Festigkeit der granulierten oder pulverisierten erfindungsgemäßen Zusammensetzung oder bei flüssigen Zusammensetzungen eine erwünschte Viskosität reproduzierbar einstellen.

Wird hingegen in der erfindungsgemäßen Zusammensetzung als Stabilisator der zuvor beschriebene Getreideextrakt bzw. der Extrakt der Samenschalen und/oder der Aleuronschicht von Getreide eingesetzt, so variiert bei dieser Ausführungsform der erfindungsgemäßen Zusammensetzung das Gewichtsverhältnis von dem mindestens einen Phospholipid zu dem zuvor genannten Stabilisator zwischen 9:0,02 und 1:0,08, vorzugsweise zwischen 6:0,02 und 1:0,04. Durch Variation der Menge an Stabilisator läßt sich auch bei dieser Ausführungsform der erfindungsgemäßen Zusammensetzung eine gewünschte Lagerstabilität einstellen, wobei bei einer gewünschten extrem langen Lagerzeit die Menge des Stabilisators im Rahmen der zuvor genannten Werte entsprechend erhöht ist.

Zu den in der erfindungsgemäßen Zusammensetzung enthaltenen Phospholipid ist festzuhalten, daß es sich hierbei insbesondere um ein aus Pflanzen und vorzugsweise aus Soja-Bohnen isoliertes Phospholipid oder Phospholipidgemisch handelt.

Besonders geeignet ist es, wenn das in der erfindungsgemäßen Zusammensetzung vorhandene Phospholipidgemisch wenigstens 70 Gew.% 1,2-Diacylglycero-3-Phosphatidylcholin aufweist.

Bei einer weiteren Ausführungsform der erfindungsgemäßen Zusammensetzung umfaßt das in der Zusammensetzung enthaltene Phospholipidgemisch mindestens 76 Gew.% ± 3 Gew.% 1,2-Diacylglycero-3-Phosphatidylcholin und 3 Gew.% ± 3 Gew.% Lysophosphatidylcholin.

Eine andere, ebenfalls geeignete Ausführungsvariante der erfindungsgemäßen Zusammensetzung sieht vor, daß hierbei das Phospholipidgemisch ein phosphatidylcholinreiches Phospholipidgemisch ist und vorzugsweise 93 Gew.% ± 3 Gew.% 1,2-Diacylglycero-3-Phosphatidylcholin und insbesondere mindestens 96 Gew.% 1,2-Diacylglycero-3-Phosphatidylcholin enthält, wobei als weiteres Phospholipid dann zusätzlich insbesondere noch 3 Gew.% ± 3 Gew.% Lyso-Phosphatidylcholin vorhanden ist.

Neben den zuvor beschriebenen Phosphatidylcholinen kann die erfindungsgemäße Zusammensetzung insbesondere noch 1,2-Diacylglycero-3-phosphoethanolamin, 1,2-Diacylglycero-3-phosphoinositol, 1,2-Diacylglycero-3-phosphoserin, 1,2-Diacylglycero-3-phosphoglycerol und 1,2-Diacylglycero-3-phosphat als weitere Phospholipide, abhängig von dem jeweiligen Ausgangsmaterial und den angewandten Isolierungs- und Reinigungsverfahren, enthalten.

Bei einer weiteren bevorzugten Ausführungsform weist die erfindungsgemäße Zusammensetzung ein solches Phospholipidgemisch auf, bei dem die Acylreste der im Gemisch enthaltenen Phospholipide und insbesondere die Acylreste des im Gemisch vorgesehenen Phosphatidylcholins zu
61 - 73 Gew.% aus dem Linolsäurerest,
10 - 14 Gew.% aus dem Palmitinsäurerest,
8 - 12 Gew.% aus dem Ölsäurerest,
4 - 6 Gew.% aus dem Linolensäurerest,
3 - 5 Gew.% aus dem Stearinsäurerest und/oder
2 Gew.% aus anderen Fettsäureresten
bestehen. Bei einem derartigen oder den nachfolgend noch konkret beschriebenen Phospholipidgemisch bzw. Phosphatidylcholinen treten insbesondere die vorstehend bei der erfindungsgemäßen Zusammensetzung beschriebenen Vorteile besonders hervor, da derartige Phospholipide bzw. Phosphatidylcholine aufgrund ihres hohen Anteils an ungesättigten Fettsäureresten selbst bei hoher Reinheit sehr schnell dazu neigen, bei Lagerung eine wachsartige Konsistenz einzunehmen und selbst bei Ausschluß von Luftsauerstoff schnell einen in der Intensität zunehmenden Eigengeruch entwickeln.

Bei einer weiteren Ausführungsform der erfindungsgemäßen Zusammensetzung weist diese ein Phospholipidgemisch bzw. ein Phosphatidylcholin auf, bei dem die 1-Acylreste der im Gemisch enthaltenen Phospholipide bzw. die 1-Acylreste des Phosphatidylcholins zu
45 - 61 Gew.% aus dem Linolsäurerest,
19 - 26 Gew.% aus dem Palmitinsäurerest,
8 - 12 Gew.% aus dem Ölsäurerest,
4 - 6 Gew.% aus dem Linolensäurerest,
6 - 9 Gew.% aus dem Stearinsäurerest und/oder
2 Gew.% aus anderen Fettsäureresten
bestehen.

Ebenso kann die erfindungsgemäße Zusammensetzung auch ein solches Phospholipidgemisch bzw. Phosphatidylcholin aufweisen, dessen Acylreste in der 1-Stellung die zuvor angegebenen Acylreste oder andere Acylreste aufweist und bei denen die Acylreste in der 2-Stellung zu
77 - 85 Gew.% aus dem Linolsäurerest,
1 - 2 Gew.% aus dem Palmitinsäurerest,
8 - 12 Gew.% aus dem Ölsäurerest,
4 - 6 Gew.% aus dem Linolensäurerest,
0 - 1 Gew.% aus dem Stearinsäurerest und/oder
2 Gew.% aus anderen Fettsäureresten
bestehen.

Die vorliegende Erfindung betrifft desweiteren ein Verfahren zur Herstellung der zuvor beschriebenen erfindungsgemäßen Zusammensetzung.

Diese Aufgabe wird erfindungsgemäß durch ein Verfahren mit den kennzeichnenden Merkmalen des Patentanspruchs 14 oder mit den kennzeichnenden Merkmalen des Patentanspruchs 21 gelöst.

Das erfindungsgemäße Verfahren zur Herstellung der zuvor beschriebenen erfindungsgemäßen phospholipidischen Zusammensetzung sieht vor, daß das mindestens eine Phospholipid bzw. das Phospholipidgemisch in einem Lösungsmittel oder Lösungsmittelgemisch zunächst gelöst oder dispergiert wird. Anschließend wird diese Lösung bzw. diese Dispersion des Phospholipids bzw. des Phospholipidgemisches in eine Lösung oder Dispersion des zuvor beschriebenen Stabilisators eingebracht, so daß dann die so hergestellte Mischung unter Ausbildung der erfindungsgemäßen phospholipidischen Zusammensetzung schonend getrocknet, insbesondere sprühgetrocknet oder gefriergetrocknet, wird.

Das erfindungsgemäße Verfahren weist den Vorteil auf, daß es relativ einfach durchzuführen ist. Dies hängt damit zusammen, daß bei dem erfindungsgemäßen Verfahren lediglich zwei Lösungen bzw. Dispersionen miteinander vermischt werden, so daß hiernach nur noch diese Mischung getrocknet wird und somit die erfindungsgemäße Zusammensetzung in entsprechender granulierter Form anfällt. Selbstverständlich ist es bei dem zuvor beschriebenen erfindungsgemäßen Verfahren auch möglich, die Lösung oder Dispersion des Stabilisators in die Lösung bzw. Dispersion des Phospholipids bzw. des Phospholipidgemischs einzubringen und hiernach diese Mischung dann schonend zu trocknen, insbesondere sprühzutrocknen oder gefrierzutrocknen.

Grundsätzlich ist bezüglich der Auswahl des bei dem erfindungsgemäßen Verfahren eingesetzten Lösungsmittels bzw. Lösungsmittelgemischs festzuhalten, daß das Lösungsmittel bzw. Lösungsmittelgemisch für das Phospholipid bzw. für das Phospholipidgemisch und das Lösungsmittel bzw. das Lösungsmittelgemisch für den Stabilisator entsprechend derart aufeinander angepaßt werden, daß diese beiden Lösungsmittel bzw. diese beiden Lösungsmittelgemische auch untereinander mischbar sind. Vorzugsweise werden bei dem erfindungsgemäßen Verfahren als Lösungsmittel bzw. Lösungsmittelgemisch Wasser und/oder ein Alkohol bzw. ein Alkoholgemisch, insbesondere Ethanol, Propanol-1 und/oder Propanol-2, verwendet.

Eine besonders geeignete und vorteilhafte Ausführungsform des erfindungsgemäßen Verfahrens sieht vor, daß hierbei eine alkoholische Lösung des Phospholipids bzw. des Phospholipidgemisches, insbesondere eine ethanolische Lösung des Phospholipids bzw. Phospholipidgemischs, hergestellt wird und daß dann diese alkoholische Lösung in eine wäßrige Dispersion oder in eine wäßrige Lösung des Stabilisators eingerührt oder injiziert wird. Unter Wasser im Rahmen der vorliegenden Beschreibung werden alle wäßrigen System verstanden, so insbesondere destilliertes Wasser, entionisiertes Wasser, wäßrige Salzlösungen oder wäßrige Puffersysteme, so beispielsweise Phosphatpuffer.

Bezüglich der Konzentration des Phospholipids bzw. des Phospholipidgemisches in der alkoholischen Lösung ist festzuhalten, daß üblicherweise bei dem erfindungsgemäßen Verfahren diese alkoholische Lösung zwischen 70 Gew.% und 85 Gew.% des Phospholipids bzw. des Phospholipidgemisches enthält. Dementsprechend weist diese alkoholische Phospholipidlösung dann zwischen 30 Gew.% und 15 Gew.% Alkohol, insbesondere Ethanol, auf.

Eine andere Ausführungsform des erfindungsgemäßen Verfahrens sieht vor, daß hierbei eine wäßrige Dispersion des Phospholipids hergestellt wird und daß diese wäßrige Dispersion des Phospholipids dann in die wäßrige Dispersion des Stabilisators eingerührt oder injiziert wird. Diese Variante des erfindungsgemäßen Verfahrens weist den Vorteil auf, daß völlig auf Alkohole als Lösungsmittel verzichtet wird, so daß diese Variante des erfindungsgemäßen Verfahrens dann bevorzugt wird, wenn der entsprechende Hersteller über keine dementsprechenden Anlagen verfügt, die die Verarbeitung von brennbaren Lösungsmittel gestattet.

Vorzugsweise enthält die zuvor beschriebene wäßrige Dispersion zwischen 5 Gew.% und 20 Gew.%, insbesondere zwischen 8 Gew.% und 15 Gew.%, des Phospholipids bzw. des Phospholipidgemisches.

Wird bei dem erfindungsgemäßen Verfahren mit einem Stabilisator gearbeitet, der die zerkleinerten Bestandteile der Samenschalen und/oder der Aleuronschicht des Getreides enthält und der, wie bereits vorstehend ausgeführt ist, somit allgemein auch als zerkleinerte Kleie zu bezeichnen ist, so empfiehlt es sich, daß hierbei die wäßrige Dispersion des Stabilisators zwischen 10 Gew.% und 30 Gew.%, insbesondere zwischen 15 Gew.% und 20 Gew.% dieses Stabilisators enthält.

Sollen bei den zuvor beschriebenen Ausführungsvarianten des erfindungsgemäßen Verfahrens noch weitere Substanzen, so insbesondere Öle, hinzugefügt werden, so daß dementsprechend die so hergestellte erfindungsgemäße Zusammensetzung dann diese weiteren Substanzen enthält, so kann dies grundsätzlich auf drei unterschiedlichen Wegen bewerkstelligt werden.

So sieht die erste Möglichkeit vor, daß hierbei diese weiteren Substanzen der Lösung bzw. der Dispersion des Phospholipids zugesetzt werden, während bei der zweiten Möglichkeit diese weiteren Substanzen zu der Lösung bzw. der Dispersion des Stabilisators zugegeben werden. Ebenso kann als dritte Möglichkeit eine separate Lösung bzw. Dispersion dieser weiteren Substanzen hergestellt werden, wobei diese Lösung bzw. Dispersion dann der Mischung aus der Phospholipidlösung bzw. der Phospholipiddispersion und der Stabilisatorlösung bzw. Stabilisatordispersion zugesetzt wird.

Eine weitere Möglichkeit zur Herstellung der erfindungsgemäßen Zusammensetzung sieht vor, daß hierbei ein flüssiges Phospholipid bzw. ein flüssiges Phospholipidgemisch hergestellt wird, derart, daß das Phospholipid bzw. das Phospholipidgemisch in einem Öl oder einem Ölgemisch aufgenommen wird. Anschließend wird das in dem Öl aufgenommen Phospholipid bzw. Phospholipidgemisch im Wirbelbett mit dem Stabilisator vermischt, wobei der feste, vorstehend bei der erfindungsgemäßen Zusammensetzung beschriebene Stabilisator diese Wirbelschicht ausbildet.

Selbstverständlich ist es möglich, in Abwandlung dieses zuvor beschriebenen Verfahrens das Phospholipid bzw. Phospholipidgemisch dadurch zu verflüssigen, daß man es nicht in einem Öl aufnimmt sondern stattdessen das Phospholipid bzw. Phospholipidgemisch selbst durch Anwendung erhöhter Temperaturen aufschmilzt und das so aufgeschmolzene Phospholipid bzw. Phospholipidgemisch in einer aus dem festen Stabilisator gebildeten Wirbelschicht mit dem Stabilisator vermischt.

Abhängig von der gewünschten weiteren Verwendung der erfindungsgemäßen Zusammensetzung wird die so nach den zuvor beschriebenen Verfahren hergestellte Zusammensetzung entweder direkt als Granulat verwendet. Hierbei variiert die Korngröße dieses Granulates zwischen etwa 0,01 mm und etwa 4 mm. Ist jedoch eine derartige granulierte Form der erfindungsgemäßen Zusammensetzung bezüglich der Korngröße unerwünscht, so sieht eine Weiterbildung des erfindungsgemäßen Verfahrens vor, daß die granulierte erfindungsgemäße Zusammensetzung anschließend über geeignete Mahlvorrichtungen zu einem Pulver zerkleinert wird, wobei die Partikelgröße dieses Pulvers vorzugsweise zwischen 0,18 mm und 0,04 mm, insbesondere zwischen 0,15 mm und 0,07, liegt.

Bezüglich der Verwendung der erfindungsgemäßen Zusammensetzung ist festzuhalten, daß die erfindungsgemäße Zusammensetzung insbesondere zur Herstellung von kosmetischen Mitteln oder pharmazeutischen Mitteln verwendet wird. Bei den kosmetischen Mitteln kann die erfindungsgemäße Zusammensetzung vorzugsweise zur Herstellung von Hautbehandlungsmitteln, insbesondere den üblichen Salben, Cremes, Lotion, Milch, Salbengrundstoffe und/oder Cremegrundstoffe verwendet werden.

Im pharmazeutischen Bereich liegt insbesondere das Hauptanwendungsgebiet der erfindungsgemäßen Zusammensetzung in der Herstellung von Diätetika, wobei derartige Diätetika dann entweder lediglich die zuvor beschriebenen Phospholipide und die zuvor genannten Stabilisatoren oder die vorstehend beschriebenen Phospholipide, die dort genannten Stabilisatoren und zusätzlich noch tierische und insbesondere pflanzliche Öle, so insbesondere Sonnenblumenöl, Distelöl, Avocadoöl, Mandelöl, Sojaöl, Rhizinusöl, Erdnußöl, Weizenkeimöl, Karottenöl, Haselnußöl, Palmkernöl, Sesamöl, Olivenöl, Walnußöl und/oder Maiskeimöl, enthält. Diese Öle liegen in der erfindungsgemäßen Zusammensetzung bei dieser Ausführungsform insbesondere in einer Konzentration zwischen 5 Gew.% und 30 Gew.%, vorzugsweise zwischen 10 Gew.% und 20 Gew.%, vor. Überraschend hat sich hierbei gezeigt, daß die zuvor genanten Diätetika eine hohe Lagerstabilität besitzen und somit selbst bei einer monatelanger Lagerung keinen störenden Eigengeruch entwickeln. Bedingt dadurch, daß vorzugsweise derartige Diätetika dann als Stabilisator zerkleinerte Bestandteile der Samenschalen und/oder Aleuronschicht des Getreides aufweisen, beinhalten derartige diätetische Mittel einen hohen Anteil an Ballaststoffen, so daß eine Sättigung bereits unter Zufuhr von wenigen Kalorien erfolgt. Die in derartigen diätetischen Mitteln enthaltenen Phospholipide erleichtern zudem noch die Anwendung, da sie einen unangenehmen Zerfall der Mittel im Mund verhindern und desweiteren den Schluckvorgang fördern. Ferner beeinflussen diese Phospholipide den Fettstoffwechsel positiv und schützen die Leber gegen die heutigen Umweltgifte.

Vorteilhafte Weiterbildungen der erfindungsgemäßen Zusammensetzung sowie des erfindungsgemäßen Verfahrens sind in den Unteransprüchen angegeben.

Die erfindungsgemäße Zusammensetzung und das erfindungsgemäße Verfahren werden nachfolgend anhand von Ausführungsbeispielen näher erläutert.

### Ausführungsbeispiel 1

800 g eines Phospholipids, das mindestens 96 Gew.% Phosphatidylcholin sowie andere Begleitphospholipide enthält, wurden in 200 g Alkohol gelöst. Die so hergestellte Lösung wurde als Lösung 1 bezeichnet.

200 g von zerkleinerten Bestandteilen der Samenschalen sowie der Aleuronschicht von Weizen (Kleie) wurden in 800 g Wasser dispergiert. Die so hergestellte Dispersion wurde als Dispersion 1 bezeichnet.

100 g der Lösung 1 wurden mit 100 g der Dispersion 1 vermischt, wobei die Vermischung während 5 Minuten mit Hilfe eines schnell laufenden Rührwerkes (1.000 U/min) erfolgte. Anschließend wurde die entstandene flüssige Mischung einer üblichen Sprühtrocknung unterworfen.

Hierbei entstand eine granulierte Zusammensetzung I, bei der das Gewichtsverhältnis von Phospholipid zu Stabilisator 4:1 betrug.

### Ausführungsbeispiel 2

50 g der Lösung 1 wurden mit 100 g der Dispersion 1, hergestellt jeweils nach dem Ausführungsbeispiel 1, unter den dort genannten Bedingungen vermischt.

Die so entstandene granulierte Zusammensetzung wies nach Sprühtrocknung ein Gewichtsverhältnis von Phospholipid zu Stabilisator von 2:1 auf.

### Ausführungsbeispiel 3

50 g der Lösung 1 wurden mit 400 g der Dispersion 1, hergestellt nach Ausführungsbeispiel 1, unter den dort genannten Bedingungen vermischt. Nach der Sprühtrocknung wies die so hergestellte granulierte Zusammensetzung III ein Gewichtsverhältnis von Phospholipid zu Stabilisator von 1:2 auf.

### Ausführungsbeispiel 4

50 g der Lösung 1 wurden mit 800 g der Dispersion 1 unter den unter dem Ausführungsbeispiel 1 genannten Bedingungen vermischt. Nach der Sprühtrocknung wies die granulierte Zusammensetzung IV ein Mischungsverhältnis von Phospholipid zu Stabilisator von 1:8 auf.

### Ausführungsbeispiel 5

50 g der Lösung 1 wurden mit 100 g der Dispersion 1 unter den im Ausführungsbeispiel 1 genannten Bedingungen vermischt. Zu der Mischung wurde unter Rühren 3 g eines Sonnenblumenöls getropft, wobei die hierbei entstehende Dispersion/Emulsion für weitere 10 Minuten gerührt wurde. Die so entstandene Mischung wurde sprühgetrocknet. Hiernach entstand eine granulierte Zusammensetzung, die nachfolgend auch als Zusammensetzung V bezeichnet ist.

### Ausführungsbeispiel 6

80 g des im Ausführungsbeispiel 1 konkretisierten Phospholipids wurden in 20 g Ethanol gelöst. In diese alkoholische Lösung wurden 1 g Tocopherol eingerührt. Anschließend erfolgte eine Sprühtrocknung. Die hierbei anfallende Zusammensetzung VI wies eine schmierige, wachsartige Konsistenz auf.

Von den vorstehend genannten Zusammensetzungen I bis VI wurde ein Alterungstest dahingehend durchgeführt, daß der Eigengeruch der Zusammensetzungen von vier Personen unabhängig voneinander sowohl unmittelbar nach der Herstellung sowie nach einer Lagerung von einer Woche, einem Monat, drei Monaten und sechs Monaten beurteilt wurde. Zur Objektivierung wurde bei dieser sensorischen Geruchsbeurteilung jeweils im Vergleich eine frisch zubereitete Probe der jeweiligen Zusammensetzung mitbeurteilt. Die Geruchsbildung wurde nach einer willkürlichen Klassifizierung, die die Noten 1 bis 4 umfaßt, vorgenommen, wobei die Note 1 keine Geruchsveränderung und die Note 4 einen starken, ranzigen Geruchseindruck wiedergibt. Die Note 2 bewertet eine geringe Geruchsveränderung, die Note 3 eine entsprechend stärkere Geruchsveränderung.

Die Ergebnisse dieser sensorischen Geruchsprüfung sind in der nachfolgenden Tabelle wiedergegeben.

**Tabelle 1**

| Zusammensetzung Nr. | unmittelbar nach der Herstellung | nach einer Woche | nach einem Monat | nach drei Monaten | nach sechs Monaten |
|---|---|---|---|---|---|
| I | 1 | 1 | 1 | 2 | 2 |
| II | 1 | 1 | 1 | 1 | 1 |
| III | 1 | 1 | 1 | 1 | 1 |
| IV | 1 | 1 | 1 | 1 | 1 |
| V | 1 | 1 | 1 | 1 | 1 |
| VI | 1 | 1 | 2 | 3 | 3 |

## Patentansprüche

1. Phospholipidische Zusammensetzung, umfassend mindestens ein Phospholipid sowie einen Stabilisator, **dadurch gekennzeichnet, daß** als Stabilisator in der phospholipidischen Zusammensetzung zerkleinerte Bestandteile und/oder ein Extrakt von Getreide enthalten sind, daß in der Zusammensetzung das Gewichtsverhältnis des mindestens einen Phospholipids zu dem Getreideextrakt zwischen 9:0,02 und 1:0,08 und/oder das Gewichtsverhältnis des mindestens einen Phospholipids zu den zerkleinerten Bestandteilen des Getreides zwischen 9:1 und 1:4 variiert und daß die Zusammensetzung ein Phospholipidgemisch enthält, das wenigstens 70 Gew.% 1,2-Diacylglycero-3-Phosphatidylcholin aufweist.

2. Zusammensetzung nach Anspruch 1, **dadurch gekennzeichnet, daß** die Zusammensetzung als Stabilisator zerkleinerte Bestandteile und/oder einen Extrakt der Samenschalen und/oder der Aleuronschicht von Getreide enthält.

3. Zusammensetzung nach Anspruch 1 oder 2, **dadurch gekennzeichnet, daß** die Zusammensetzung zerkleinerte Bestandteile und/oder den Extrakt der Samenschalen und/oder der Aleuronschicht von Weizen, Hafer, Mais, Reis, Roggen, Gerste und/oder Hirse aufweist.

4. Zusammensetzung nach einem der vorangehenden Ansprüche, **dadurch gekennzeichnet, daß** in der Zusammensetzung das Gewichtsverhältnis von dem mindestens einen Phospholipid zu den zerkleinerten Bestandteilen des Getreides zwischen 6:1 und 1:2 variiert.

5. Zusammensetzung nach einem der vorangehenden Ansprüche, **dadurch gekennzeichnet, daß** in der Zusammensetzung das Gewichtsverhältnis von dem mindestens einen Phospholipid zu dem Getreideextrakt zwischen 6:0,02 und 1:0,04, variiert.

6. Zusammensetzung nach einem der vorangehenden Ansprüche, **dadurch gekennzeichnet, daß** das Phospholipid aus Pflanzen, vorzugsweise aus Soja-Bohnen, isoliert ist.

7. Zusammensetzung nach einem der vorangehenden Ansprüche, **dadurch gekennzeichnet, daß** das mindestens eine Phospholipid ein Phospholipidgemisch ist.

8. Zusammensetzung nach einem der vorangehenden Ansprüche, **dadurch gekennzeichnet, daß** das Phospholipidgemisch mindestens
76 Gew.% ± 3 Gew.% 1,2-Diacylglycero-3-Phosphatidylcholin und
3 Gew.% ± 3 Gew.% Lyso-Phosphatidylcholin
enthält.

9. Zusammensetzung nach einem der vorangehenden Ansprüche, **dadurch gekennzeichnet, daß** das Phospholipidgemisch mindestens
93 Gew.-% ± 3 Gew.% 1,2-Diacylglycero-3-Phosphatidylcholin und
3 Gew.% ± 3 Gew.% Lyso-Phosphatidylcholin
enthält.

10. Zusammensetzung nach einem der vorangehenden Ansprüche, **dadurch gekennzeichnet, daß** das Phospholipidgemisch desweiteren
1, 2-Diacylglycero-3-phosphoethanolamin,
1,2-Diacylglycero-3-phosphoinositol,
1,2-Diacylglycero-3-phosphoserin,
1,2-Diacylglycero-3-phosphoglycerol und/oder
1,2-Diacylglycero-3-phosphat
enthält.

11. Zusammensetzung nach einem der vorangehenden Ansprüche, **dadurch gekennzeichnet, daß** die Zusammensetzung ein solches Phospholipidgemisch enthält, bei dem die Acylreste der im Gemisch enthaltenen Phospholipide zu
61 - 73 Gew.% aus dem Linolsäurerest,
10 - 14 Gew.% aus dem Palmitinsäurerest,
8 - 12 Gew.% aus dem Ölsäurerest,
4 - 6 Gew.% aus dem Linolensäurerest,
3 - 5 Gew.% aus dem Stearinsäurerest und/oder
2 Gew.% aus anderen Fettsäureresten
bestehen.

12. Zusammensetzung nach einem der vorangehenden Ansprüche, **dadurch gekennzeichnet, daß** die Zusammensetzung ein solches Phospholipidgemisch enthält, bei dem die 1-Acylreste der im Gemisch enthaltenen Phospholipide zu
45 - 61 Gew.% aus dem Linolsäurerest,
19 - 26 Gew.% aus dem Palmitinsäurerest,
8 - 12 Gew.% aus dem Ölsäurerest,
4 - 6 Gew.% aus dem Linolensäurerest,
6 - 9 Gew.% aus dem Stearinsäurerest und/oder
2 Gew.% aus anderen Fettsäureresten
bestehen.

13. Zusammensetzung nach einem der vorangehenden Ansprüche, **dadurch gekennzeichnet, daß** die Zusammensetzung ein solches Phospholipidgemisch enthält, bei dem die 2-Acylreste der im Gemisch enthaltenen Phospholipide zu
77 - 85 Gew.% aus dem Linolsäurerest,
1 - 2 Gew.% aus dem Palmitinsäurerest,
8 - 12 Gew.% aus dem Ölsäurerest,
4 - 6 Gew.% aus dem Linolensäurerest,
0 - 1 Gew.% aus dem Stearinsäurerest und/oder
2 Gew.% aus anderen Fettsäureresten
bestehen.

14. Verfahren zur Herstellung einer phospholipidischen Zusammensetzung nach einem der vorangehenden Ansprüche, **dadurch gekennzeichnet, daß** als Stabilisator in der phospholipidischen Zusammensetzung zerkleinerte Bestandteile und/oder ein Extrakt von Getreide ausgewählt werden, daß in der Zusammensetzung das Gewichtsverhältnis des mindestens einen Phospholipids zu dem Getreideextrakt zwischen 9:0,02 und 1:0,08 und/oder das Gewichtsverhältnis des mindestens einen Phospholipids zu den zerkleinerten Bestandteilen des Getreides zwischen 9:1 und 1:4 variiert wird, daß in der Zusammensetzung ein Phospholipidgemisch vorgesehen wird, das wenigstens 70 Gew.% 1,2-Diacylglycero-3-Phosphatidylcholin aufweist, daß das mindestens eine Phospholipid in einem Lösungsmittel oder Lösungsmittelgemisch gelöst oder dispergiert wird, daß die Lösung bzw. Dispersion des Phospholipids in eine Lösung oder Dispersion des Stabilisators eingebracht wird und daß die so hergestellte Mischung anschließend schonend getrocknet, insbesondere sprühgetrocknet oder gefriergetrocknet, wird.

15. Verfahren nach Anspruch 14, **dadurch gekennzeichnet, daß** als Lösungsmittel bzw. Lösungsmittelgemisch Wasser und/oder ein Alkohol, vorzugsweise Ethanol, Propanol-1 und/oder Propanol-2, verwendet wird.

16. Verfahren nach Anspruch 14 oder 15, **dadurch gekennzeichnet, daß** eine alkoholische Lösung des Phospholipids hergestellt wird und daß die alkoholische Lösung in eine wäßrige Dispersion oder in eine wäßrige Lösung des Stabilisators eingerührt oder injiziert wird.

17. Verfahren nach Anspruch 16, **dadurch gekennzeichnet, daß** die alkoholische Phospholipidlösung 70 Gew.% bis 85 Gew.% Phospholipid und 30 Gew.% bis 15 Gew.% des Alkohol enthält.

18. Verfahren nach Anspruch 14 oder 15, **dadurch gekennzeichnet, daß** eine wäßrige Dispersion des Phospholipids hergestellt wird und daß die wäßrige Dispersion in die wäßrige Dispersion des Stabilisators eingerührt oder injiziert wird.

19. Verfahren nach.Anspruch 18, **dadurch gekennzeichnet, daß** die wäßrige Dispersion 5 Gew.% bis 20 Gew.%, insbesondere 8 Gew.% bis 15 Gew.%, des Phospholipids enthält.

20. Verfahren nach einem der Ansprüche 14 bis 19, **dadurch gekennzeichnet, daß** als Stabilisator zerkleinerte Bestandteile der Samenschalen und/oder der Aleuronschicht des Getreides verwendet wird und daß die wäßrige Dispersion 10 Gew.% bis 30 Gew.% des Stabilisators enthält.

21. Verfahren zur Herstellung der Zusammensetzung nach einem der Ansprüche 1 bis 13, **dadurch gekennzeichnet, daß** ein flüssiges Phospholipid bzw. Phospholipidgemisch hergestellt wird, derart, daß das Phospholipid bzw. Phospholipidgemisch in einem Öl aufgenommen wird, und daß das in dem Öl aufgenommene Phospholipid bzw. Phospholipidgemisch dann im Wirbelbett mit dem Stabilisator vermischt wird.

22. Verfahren nach einem der Ansprüche 14 bis 20, **dadurch gekennzeichnet, daß** die phospholipidische Zusammensetzung nach der Trocknung auf eine Korngröße zwischen 0,18 mm und 0,04 mm vermahlen wird.

23. Verwendung der Zusammensetzung nach einem der vorangehenden Ansprüche zur Herstellung von kosmetischen Mitteln.

24. Verwendung der Zusammensetzung nach einem der vorangehenden Ansprüche zur Herstellung von pharmazeutischen Mitteln.

25. Verwendung nach Anspruch 24 als diätetische Zubereitung.

## Claims

1. A phospholipidic composition comprising at least a phospholipide as well as a stabilizer, **characterized in that** along with the at least one phospholipide the composition furthermore comprises as a stabilizer ground parts and/or an extract of grain, that within the composition the weight ratio of the at least one phospholipide to the extract of the grain varies between 9:0,02 and 1:0,08 and/or that the weight ratio of the at least one phospholipide to the ground parts of the grain varies between 9:1 and that the composition comprises a mixture of phospholipides, which contains at least 70 % by weight of 1.2-diacylglycero-3-phosphatidylcholine.

2. The composition according to claim 1, **characterized in that** the composition comprises as a stabilizer ground parts and/or an extract of the seed husks and/or of the aleurone layer of grain.

3. The composition according to claim 1 or 2, **characterized in that** the composition contains ground parts and/or an extract of the seed husks and/or of the aleurone layer of wheat, oats, corn, rice, rye, barley and/or millet.

4. The composition according to one of the proceeding claims **characterized in that** the weight ratio of the at least one phospholipide to the ground parts of the grain varies between 6:1 and 1:2 in the composition.

5. The composition according to one of the proceeding claims **characterized in that** the weight ratio of the at least one phospholipide to the extract of the grain varies between 6:0,02 and 1:0,04 in the composition.

6. The composition according to one of the proceeding claims **characterized in that** the phospholipide is isolated of plants, preferably of soy beans.

7. The composition according to one of the proceeding claims **characterized in that** the least one phospholipide is a mixture of phospholipids.

8. The composition according to one of the proceeding claims **characterized in that** the mixture of phospholipides comprises at least
76 % by weight ± 3 % by weight of 1.2-diacylglycero-3-phosphatidylcholine, and
3 % by weight ± 3 % by weight of lyso-phosphatidylcholine.

9. The composition according to one of the proceeding claims **characterized in that** the mixture of phospholipides comprises at least
93 % by weight ± 3 % by weight of 1.2-diacylglycero-3-phosphatidylcholine, and
3 % by weight ± 3 % by weight of lyso-phosphatidylcholine.

10. The composition according to one of the proceeding claims **characterized in that** the mixture
of phospholipides comprises furthermore
1.2-diacylglycero-3-phosphoethanolamine,
1.2-diacylglycero-3-phosphoinositol,
1.2-diacylglycero-3-phosphoserine,
1.2-diacylglycero-3-phosphoglycerol, and/or
1.2-diacylglycero-3-phosphate

11. The composition according to one of the proceeding claims **characterized in that** the composition comprises such a mixture of phospholipides, with which the acyl groups of the phospholipides contained in the mixture consist of
61 - 73 % by weight of the linoleic acid residue,
10 - 14 % by weight of the palmitic acid residue,
8 - 12 % by weight of the oleic acid residue,
4 - 6 % by weight of the linolenic acid residue,
3 - 5 % by weight of the stearic acid residue, and/or
2 % by weight of other fatty acid residues.

12. The composition according to one of the proceeding claims **characterized in that** the composition comprises such a mixture of phospholipides, with which the 1-acyl groups of the phospholipides contained in the mixture consist of
45 - 61 % by weight of the linoleic acid residue,
19 - 26 % by weight of the palmitic acid residue,
8 - 12 % by weight of the oleic acid residue,
4 - 6 % by weight of the linolenic acid residue,
6 - 9 % by weight of the stearic acid residue, and/or
2 % by weight of other fatty acid residues.

13. The composition according to one of the proceeding claims **characterized in that** the composition comprises such a mixture of phospholipides, with which the 2-acyl groups of the phospholipides contained in the mixture consist of
77 - 85 % by weight of the linoleic acid residue,
1 - 2 % by weight of the palmitic acid residue,
8 - 12 % by weight of the oleic acid residue,
4 - 6 % by weight of the linolenic acid residue,
0 - 1 % by weight of the stearic acid residue, and/or
2 % by weight of other fatty acid residues.

14. A method for the production of a phospholipidic composition according to one of the proceeding claims **characterized in that** within the phospholipidic composition as stabilizer ground parts and/or an extract of grain are chosen, that within the composition the weight ratio of the at least one phospholipide to the extract of the grain is varied between 9:0,02 and 1:0,08 and/or that the weight ratio of the at least one phospholipide to the ground parts of the grain is varied between 9:1 and 1:4, that within the composition a mixture of phospholipids is provided which contains at least 70 % by weight of 1.2-diacylglycero-3-phosphatidylcholine, that the at least one phospholipide is dissolved or dispersed in a solvent or a mixture of solvents, that the solution or dispersion of the phospholipide is inserted into a solution or dispersion of the stabilizer and that hereafter the mixture produced in such a way is carefully dried, especially spray-dried or freeze-dried.

15. The method according to claim 14 **characterized in that** water and/or an alcohol, preferably ethanol, 1-propanol and/or 2-propanol, is used as a solvent or as a mixture of solvents.

16. The method according to claim 14 or 15 **characterized in that** an alcoholic solution of the phospholipide is produced and that the alcoholic solution is stirred or injected into an aqueous dispersion or an aqueous solution of the stabilizer.

17. The method according to claim 16 **characterized in that** the alcoholic phospholipide solution comprises 70 % by weight to 85 % by weight of the phospholipide and 30 % by weight to 15 % by weight of the alcohol.

18. The method according to claim 14 or 15 **characterized in that** an aqueous dispersion of the phospholipide is produced and that the aqueous dispersion is stirred or injected into the aqueous dispersion of the stabilizer.

19. The method according to claim 18 **characterized in that** the aqueous dispersion comprises 5 % by weight to 20 % by weight, especially 8 % by weight to 15 % by weight, of the phospholipide.

20. The method according to one of the claims 14 to 19 **characterized in that** ground parts of the seed husks and/or of the aleurone layer of the grain are used and that the aqueous dispersion comprises 10 % by weight to 30 % by weight of the stabilizer.

21. The method for the production of the composition according to one of the claims 1 to 13 **characterized in that** a liquid phospholipide or mixture of phospholipides is produced in such a way, that the phospholipide or mixture of phospholipides is added to an oil, and that the phospholipide or mixture of phospholipides added to the oil, is then mixed with the stabilizer in a fluid bed.

22. The method according to one of the claims 14 to 20 **characterized in that** the phospholipidic composition is ground to a granular size between 0,18 mm and 0,04 mm after the drying.

23. The use of the composition according to one of the proceeding claims for the production of cosmetics.

24. The use of the composition according to one of the proceeding claims for the production of pharmaceutical products.

25. The use according to claim 24 as a dietary formulation.

## Revendications

1. Composition phospholipidique, comprenant au moins un phospholipide ainsi qu'un stabilisateur, **caractérisé en ce que** la composition phospholipidique contient comme stabilisateur des composants broyés et/ou un extrait de céréale, **en ce que**, dans la composition, le rapport pondéral du au moins un phospholipide à l'extrait de céréale varie entre 9:0,02 et 1:0,08 et/ou le rapport pondéral du au moins un phospholipide aux composants broyés de la céréale varie entre 9:1 et 1:4, et **en ce que** la composition contient un mélange de phospholipides, qui présente au moins 70% en poids de 1,2-diacylglycéro-3-phosphatidyl-choline.

2. Composition selon la revendication 1, **caractérisée en ce que** la composition contient comme stabilisateur des composants broyés et/ou un extrait des enveloppes de graines et/ou de la couche d'aleurone de céréales.

3. Composition selon la revendication 1 ou 2, **caractérisée en ce que** la composition présente des composants broyés et/ou l'extrait des enveloppes de graines et/ou la couche d'aleurone de blé, d'avoine, de maïs, de riz, de seigle, d'orge et/ou de millet.

4. Composition selon l'une quelconque des revendications précédentes, **caractérisée en ce que**, dans la composition, le rapport pondéral du au moins un phospholipide aux composants broyés de la céréale varie entre 6:1 et 1:2.

5. Composition selon l'une quelconque des revendications précédentes, **caractérisée en ce que**, dans la composition, le rapport pondéral du au moins un phospholipide à l'extrait de céréale varie entre 6:0,02 et 1:0,04.

6. Composition selon l'une quelconque des revendications précédentes, **caractérisée en ce que** le phospholipide est isolé de plantes, de préférence, de fèves de soja.

7. Composition selon l'une quelconque des revendications précédentes, **caractérisée en ce que** le au moins un phospholipide est un mélange de phospholipides.

8. Composition selon l'une quelconque des revendications précédentes, **caractérisée en ce que** le mélange de phospholipides contient au moins :
76% en poids ± 3% en poids de 1,2-diacylglycéro-3-phosphatidylcholine et 3% en poids ± 3% en poids de lysophosphatidylcholine.

9. Composition selon l'une quelconque des revendications précédentes, **caractérisée en ce que** le mélange de phospholipides contient au moins :
93% en poids ± 3% en poids de 1,2-diacylglycéro-3-phosphatidylcholine et 3% en poids ± 3% en poids de lysophosphatidylcholine.

10. Composition selon l'une quelconque des revendications précédentes, **caractérisée en ce que** le mélange de phospholipides contient, en outre, les substances suivantes :
- la 1,2-diacylglycéro-3-phosphoéthanolamine,
- le 1,2-diacylglycéro-3-phosphoinositol,
- la 1,2-diacylglycéro-3-phosphosérine,
- le 1,2-diacylglycéro-3-phosphoglycérol et/ou
- le 1,2-diacylglycéro-3-phosphate.

11. Composition selon l'une quelconque des revendications précédentes, **caractérisée en ce que** la composition contient un mélange de phospholipides, dans lequel les radicaux acyle des phospholipides contenus dans le mélange sont constitués de :
- 61 à 73% en poids du radical acide linoléique,
- 10 à 14% en poids du radical acide palmitique,
- 8 à 12% en poids du radical acide oléique,
- 4 à 6% en poids du radical acide linolénique,
- 3 à 5% en poids du radical acide stéarique et/ou
- 2% en poids d'autres radicaux d'acides gras.

12. Composition selon l'une quelconque des revendications précédentes, **caractérisée en ce que** la composition contient un mélange de phospholipides, dans lequel les radicaux 1-acyle des phospholipides contenus dans le mélange sont constitués de :
- 45 à 61% en poids du radical acide linoléique,
- 19 à 26% en poids du radical acide palmitique,
- 8 à 12% en poids du radical acide oléique,
- 4 à 6% en poids du radical acide linolénique,
- 6 à 9% en poids du radical acide stéarique et/ou
- 2% en poids d'autres radicaux d'acides gras.

13. Composition selon l'une quelconque des revendications précédentes, **caractérisée en ce que** la composition contient un mélange de phospholipides, dans lequel les radicaux 2-acyle des phospholipides contenus dans le mélange sont constitués de :
- 77 à 85% en poids du radical acide linoléique,
- 1 à 2% en poids du radical acide palmitique,
- 8 à 12% en poids du radical acide oléique,
- 4 à 6% en poids du radical acide linolénique,
- 0 à 1% en poids du radical acide stéarique et/ou
- 2% en poids d'autres radicaux d'acides gras.

14. Procédé de fabrication d'une composition phospholipidique selon l'une quelconque des revendications précédentes, **caractérisé en ce que** l'on choisit comme stabilisateur dans la composition phospholipidique des composants broyés et/ou un extrait de céréale, **en ce que**, dans la composition, le rapport pondéral du au moins un phospholipide à l'extrait de céréale varie entre 9:0,02 et 1:0,08 et/ou le rapport pondéral du au moins un phospholipide aux composants broyés de la céréale varie entre 9:1 et 1:4, **en ce que** la composition contient un mélange de phospholipides qui présente au moins 70% en poids de 1,2-diacylglycéro-3-phosphatidylcholine, **en ce que** le au moins un phospholipide est dispersé dans un solvant ou un mélange de solvants, **en ce que** la solution ou la dispersion du phospholipide est incorporée à une solution ou à une dispersion du stabilisateur et **en ce que** le mélange ainsi fabriqué est ensuite séché délicatement, en particulier, par pulvérisation ou par lyophilisation.

15. Procédé selon la revendication 14, **caractérisé en ce que** l'on utilise comme solvant ou mélange de solvants, l'eau et/ou un alcool, de préférence, l'éthanol, le propanol-1 et/ou le propanol-2.

16. Procédé selon la revendication 14 ou 15, **caractérisé en ce que** l'on fabrique une solution alcoolique du phospholipide et **en ce que** la solution alcoolique est mélangée à une dispersion aqueuse ou à une solution aqueuse du stabilisateur, ou y est injectée.

17. Procédé selon la revendication 16, **caractérisé en ce que** la solution alcoolique de phospholipides contient 70% en poids à 85% en poids de phospholipides et 30% en poids à 15% en poids d'alcool.

18. Procédé selon la revendication 14 ou 15, **caractérisé en ce que** l'on fabrique une dispersion aqueuse du phospholipide et **en ce que** la dispersion aqueuse est mélangée à la dispersion aqueuse du stabilisateur ou y est injectée.

19. Procédé selon la revendication 18, **caractérisé en ce que** la dispersion aqueuse contient 5% en poids à 20% en poids, en particulier, 8% en poids à 15% en poids du phospholipide.

20. Procédé selon l'une quelconque des revendications 14 à 19, **caractérisé en ce que** l'on utilise comme stabilisateur des composants broyés des enveloppes de grains et/ou de couche d'aleurone de la céréale, et **en ce que** la dispersion aqueuse contient 10% en poids à 30% en poids du stabilisateur.

21. Procédé de fabrication de la composition selon l'une quelconque des revendications 1 à 13, **caractérisé en ce que** l'on fabrique un phospholipide ou un mélange de phospholipides liquide de manière que le phospholipide ou le mélange de phospholipides soit absorbé dans une huile, et **en ce que** le phospholipide ou le mélange de phospholipides absorbé dans l'huile est ensuite mélangé au stabilisateur dans le lit fluidisé.

22. Procédé selon l'une quelconque des revendications 14 à 20, **caractérisé en ce que** la composition phospholipidique est broyée après séchage à une granulométrie comprise entre 0,18 mm et 0,04 mm.

23. Utilisation de la composition selon l'une quelconque des revendications précédentes pour fabriquer des agents cosmétiques.

24. Utilisation de la composition selon l'une quelconque des revendications précédentes pour fabriquer des agents pharmaceutiques.

25. Utilisation selon la revendication 24, comme préparation diététique.
